# EUROPÄISCHE PATENTANMELDUNG

(11) **EP 2 535 020 A1**
(43) Veröffentlichungstag der Anmeldung: **19.12.2012**
(21) Anmeldenummer: 11170134.8
(22) Anmeldetag: 16.06.2011
(51) Int. Cl.: A61F 2/38, A61F 2/00

(54) **Rotationsgelenkprothesen mit verstärkter Lagerbuchse**

(71) Anmelder: Deru GmbH, 22844 Norderstedt (DE)
(72) Erfinder: Bartels, Carolin, 25355 Barmstedt (DE); Dmuschewsky, Klaus, 22397 Hamburg (DE); Iredi, Marco, 22848 Norderstedt (DE)
(74) Vertreter: Glawe, Delfs, Moll

(57) **Zusammenfassung**

Die Erfindung betrifft eine Gelenkprothese mit einer distalen Komponente (1) zur Verankerung an einem ersten Knochen, einer proximalen Komponente (2) zur Verankerung mit einem zweiten Knochen, einem Kopplungsstück (3), das der ersten Komponente (1) ein Flexionslager um eine erste Achse (34) und mit der zweiten Komponente (2) ein aus Zapfen (31) und Lagerbuchse (32) gebildetes Rotationslager um eine quer zur ersten Achse (34) orientierte zweite Achse bildet, wobei das Rotationslager einen mehrlagigen Lagereinsatz (4) umfasst mit einer den Zapfen (31) umfassenden Gleithülse (41) und eine diese umkammernde Stützhülse (42), die über ein Sicherungselement (5) an dem Koppelstück (3) befestigt ist, wobei das Sicherungselement (5) ein Betätigungsorgan (52) innerhalb der Stützhülse (42) aufweist und über zwei fluchtende Bohrungen (47, 38) in Schutzhülse (42) und Koppelstück (3) zugfest mit dem Koppelstück (3) verbindbar ist.

## Beschreibung

Die Erfindung betrifft eine Rotationsgelenkprothese mit einer Distalkomponente und einer proximalen Komponente sowie einem sie gelenkig verbindenen Kopplungsstück, das ein Flexionslager um eine erste Achse und ein Rotationslager um eine quer zur ersten Achse orientierte zweite Achse umfasst.

Prothesen der eingangsgenannten Art finden insbesondere Verwendung als Kniegelenk beziehungsweise Ellbogenprothesen. Insbesondere Kniegelenkprothesen sind aufgrund ihrer hohen Belastung durch das Körpergewicht und ihren komplexen Bewegungsablauf, nämlich der Beugebewegung (Flexion) als Hauptbewegung kombiniert mit einer Drehung (Rotation) des Schienenbeins relativ zum Oberschenkel als Nebenbewegung vergleichsweise anfällig gegenüber Störungen durch Verschleiß oder Krankheit. Um dies zu therapieren werden Knieprothesen benötigt, welche vorzugsweise im Interesse einer möglichst anatomischen Wiederherstellung der Gelenkfunktion beide Bewegungen ermöglichen. Eine weitere Anforderung an die Gelenkprothese ist, dass sie eine ausreichende Stabilität haben muss, insbesondere auch dann, wenn der aus Weichteilen bestehender Halteapparat (Bänder etc.) bereits geschwächt ist.

Im Stand der Technik sind verschiedene Knieprothesen bekannt geworden, um diese teilweise konträren Zielsetzungen der Beweglichkeit um zwei Achsen einerseits mit der Gewährleistung einer ausreichenden Stabilität andererseits zu verknüpfen. Bei einer bekannten Rotationsknieprothese (EP 0174531 B1) sind eine tibiale Komponente und eine femorale Komponente über ein Koppelstück miteinander verbunden, welches ein Achsauge zur Aufnahme einer eine femoralen Komponente tragenden Achse aufweist, um so ein Beugegelenk zu schaffen. Weiter weist das Koppelstück eine zur Tibia hin weisende Lagerbuchse auf, in die ein an der tibialen Komponente angeordneter Zapfen eingreift, um so ein Rotationsgelenk zu bilden. Um günstige Reibverhältnisse bei diesem aus Zapfen und Lagerbuchse gebildeten Rotationslager zu schaffen, ist in der Lagerbuchse eine Gleithülse aus Kunststoff angeordnet. Sie ist hutförmig ausgebildet mit einem halbkugelförmigen, die Spitze des Zapfens bedeckenden Oberteil. Der im Wesentlichen zylindrische Mantel liegt an der die Lagerbuchse bildenden Wandung des Koppelstücks an. Damit ergibt sich ein Verkammerungseffekt. Es hat sich jedoch gezeigt, dass es dennoch zu Verquetschungen der Lagerhülse kommen kann. Für den Patienten bedeutet dieser Defekt der Prothese in der Regel eine Revisionsoperation.

Um diesem Nachteil zu begegnen ist eine unter der Bezeichnung "Endo Modell" von Waldemar Link, Hamburg, vertriebene verbesserte Kniegelenkendoprothese bekannt geworden. Bei ihr ist die Metallhülse zweiteilig ausgeführt, wobei das gleitgünstige Kunststoffmaterial eng umfasst ist von einer aufgeschrumpften und mehrfach durchbohrten Metallhülse. Durch diese Einheit ist das Kunststoffmaterial besser abgestützt, so dass das Risiko eines Defekts der Gleithülse, insbesondere aufgrund von Kaltfluss des Kunststoffes wegen der hohen Belastung, deutlich vermindert ist. In der Praxis hat sich aber gezeigt, dass nun die in Kraftflussrichtung wichtigste Komponente, nämlich das die eigentliche Lagerbuchse aufnehmende Koppelstück, anfällig gegenüber Ausfällen durch Überlastung ist. Bei einer Revision der Prothese gestattet sich häufig das Herausziehen der Lagerbuchse schwierig.

Der Erfindung liegt die Aufgabe zugrunde, eine Gelenkprothese der eingangsgenannten Art dahingehend zu verbessern, dass sie robuster und im Revisionsfall leichter zu demontieren ist.

Die erfindungsgemäße Lösung liegt in den Merkmalen des unabhängigen Anspruchs. Vorteilhafte Weiterbildungen sind Gegenstand der abhängigen Ansprüche.

Bei einer Rotationsgelenkprothese mit einer distalen Komponente zur Verankerung in einem ersten Knochen, einer proximalen Komponente zur Verankerung an einem zweiten Knochen und einem Kopplungsstück, das mit der proximalen Komponente ein Flexionslager um eine erste Achse bildet und mit der zweiten Komponente ein aus Stift und Lagerbuchse gebildetes Rotationslager quer zur ersten Achse orientierte zweite Achse bildet, und in der Lagerbuchse eine den Stift umfassende Gleithülse und eine diese vollständig umkammernde Stützhülse angeordnet ist, die über ein Sicherungselement an dem Koppelstück befestigt ist, wobei erfindungsgemäß vorgesehen ist, dass das Sicherungselement ein Betätigungsorgan innerhalb der Stützhülse aufweist und über zwei fluchtende Bohrungen in Stützhülse und Koppelstück zugfest mit dem Koppelstück verbindbar ist.

Kern der Erfindung ist der Gedanke, das Betätigungsorgan für das Sicherungselement in das Innere der Lagerbuchse zu positionieren. Anders als bei dem nächstliegenden Stand der Technik braucht dann der Zugang zum Sicherungselement nicht von außen durch das Koppelstück zu führen, wofür eine entsprechende Öffnung im Koppelstück erforderlich war, was zu dessen Schwächung geführt hat. Dank des erfindungsgemäß nach innen verlegte Betätigungsorgan kann die Öffnung am Koppelstück deutlich kleiner ausfallen, weshalb es an Stabilität gewinnt. Weiter kann damit das Sicherungselement direkt an der Lagerbuchse angreifen, so dass sie auch dann, wenn sie verhältnismäßig festsitzt, sicher herausgezogen werden kann. Damit sind die Voraussetzungen geschaffen, die Stützhülse durch dickeres Material zu verstärken und sie so in den Lastfluss gar einzubinden, was zu einer entsprechenden Entlastung der üblicherweise aus Kunststoffmaterial gefertigten Gleithülse führt. Damit können auch größere Belastungen sicher aufgenommen werden, und die Gefahr von Ausfällen aufgrund von Überlastung sinkt. Unter dicker Materialstärke der Stützhülse wird hierbei verstanden, dass die Materialstärke mindestens das 0,8-fache der Materialstärke der Gleithülse beträgt.

Vorzugsweise ist an der Gleithülse eine Zugangsöffnung zum Betätigungsorgan vorgesehen. Damit liegt das Betätigungsorgan von der Eintrittsseite des Zapfens gesehen hinter der Gleithülse, und kann diese bei dem Lösen des Sicherungselements mitnehmen. Damit wirkt das Sicherungselement mit seinem Betätigungsorgan gleichzeitig als ein Abzieher für Gleithülse. Auch beschädigte Gleithülsen, insbesondere aufgrund von Verquetschung beziehungsweise Kaltfluss des Kunststoffmaterials bei Überlastung, können damit leicht entfernt werden. Eine besonders zweckmäßige Ausbildung des Sicherungselements ist eine Schraube, deren Kopf das Betätigungsorgan bildet. Damit kann auf konstruktiv einfache Weise einerseits eine feste Sicherung im montierten Zustand erreicht werden, und andererseits kann durch Verdrehen der Schraube dank der durch das Gewinde wirkenden Kraftumsetzung eine beträchtliche abdrückende Wirkung auf die Leithülse ausgeübt werden. Hierbei ist zweckmäßigerweise die Weite der Zugangsöffnung kleiner als die Weite des Kopfes der Schrauben. Bewährt hat es sich, wenn die Zugangsöffnung der Gleithülse sich zur Stützhülse hin erweitert. Damit ergibt sich eine Konizität, die das Abdrücken begünstigt. Vorzugsweise sind Bohrungen für das Sicherungselement in der Stützhülse einerseits und dem Koppelstück andererseits mit unterschiedlicher Weite ausgeführt. Eine derart gestufte Bohrung hat den Vorteil, dass das Sicherungselement mit seinem Schaft durch die Stützhülse hindurch gesteckt werden kann, bis es mit seinem Kopf aufliegt. Bei der Schraube eines Sicherungselements bedeutet dies, dass der Durchmesser der Bohrung in der Stützhülse größer ist als der Schaftdurchmesser der Schraube, aber kleiner als der Durchmesser des Kopfes, und dass an dem Koppelstück die Bohrung einen weiter entsprechenden Kerndurchmesser der Schraube aufweist, und an der Bohrung ein Innengewinde ausgeführt ist. Damit kann durch Festziehen des Sicherungselements, insbesondere durch Festziehen der Schraube, eine sichere Positionierung erreicht werden, wobei durch Lösen nicht nur die Sicherung aufgehoben wird, sondern auch die Gleithülse herausgedrückt werden kann.

Um im Fall einer festsitzenden Stützhülse auch diese bei einer Revision leicht und sicher entfernen zu können, ist vorzugsweise an ihr eine mit der Zugangsöffnung fluchtende Aufnahme für einen Extraktor vorgesehen. Besonders bewährt hat es sich, die Aufnahme als ein Innengewinde in der Bohrung für das Sicherungselement auszuführen. Damit kann nach Entfernen des Sicherungselements durch Einbringen des Extraktors und Verbinden mit der Stützhülse diese einfach aus dem Koppelstück herausgezogen werden. Bei der Ausführung im Innengewinde braucht dazu der Extraktor nur an seiner Spitze ein entsprechendes Gegengewinde aufzuweisen, welches durch einfaches Einschrauben mit der Stützhülse verbunden wird.

Mit Vorteil ist die Gleithülse über im Bereich ihrer stiftseitigen Mündung angeordnete Rastnasen, die in einen Hinterschnitt an der Innenfläche der Stützhülse greifen, vor einer axiale Bewegung aus der Stützhülse heraus gesichert. Einer Beschädigung durch Luxationsbewegung wird damit entgegengewirkt.

Mit Vorteil ist die Stützhülse mit einer nach radial vorspringenden Leiste versehen, die in einen komplementär geformten Rezess am Koppelstück greift. Damit ist sichergestellt, dass sich die Stützhülse bei der Rotationsbewegung um den Zapfen nicht in unerwünschter Weise mitdreht. Indem die Nase und der Rezess am zapfenseitigen Ende angeordnet sind, kann die formschlüssige Verbindung durch einfaches Einschieben der Stützhülse in ihre Lagerbuchse an dem Koppelstück erreicht werden. Im Übrigen ist die Aufnahme für die Stützhülse vorzugsweise glattwandig mit insbesondere zylindrischer Innenform an dem Koppelstück ausgeführt. Dies ist einfach in der Herstellung und ergibt eine gleichmäßig stabile Lagerung, auch bei einer axialen Bewegung des Zapfens in der Lagerbuchse.

Bei einer besonders bevorzugten Ausführungsform, die gegebenenfalls unabhängigen Schutz verdient, ist an einer Vorderseite des Koppelstücks eine Aufnahmefläche für einen Prallschutz vorgesehen, der über eine taschenartige Ausnehmung in den Bereich eines Aufnahmeauges verlängert ist. Der Prallschutz fungiert als Anschlag für die Flexionsbewegung, und zwar für die gestreckte Stellung. Da hierbei das Koppelstück an der distalen Komponente anschlagen kann, dient der Prallschutz dazu, es vor einer Beschädigung zu schützen. Um bei stoßartig auftretenden Belastungen in seiner vorgesehenen Position zu verbleiben, ist eine ausreichend sichere Befestigung erforderlich. Auf einfache Weise kann mittels der taschenartigen Ausnehmung erreicht werden, dass der Prallschutz an einem Abheben und Wegfallen von seiner Position an der Vorderseite des Koppelstücks gehindert ist. Mit Vorteil weist die taschenartige Ausnehmung einen rechteckigen Querschnitt auf und geht plan in die Aufnahmefläche über. Damit kann der Prallschutz als ein durchgehendes Plättchen ausgeführt sein, welches durch Einschieben der taschenartigen Ausnehmung in seiner Position fixiert ist. Um ein unerwünschtes Herausbewegen aus der taschenartigen Ausnehmung zu vermeiden, ist vorzugsweise eine, maximal zwei Vertiefungen an der Vorderseite im Koppelstück ausgeformt, die als Verschiebesicherung besonders mit einem an der Rückseite des Prallschutzes angeordneten Vorsprung fungieren. Damit ist Prallschutz nicht nur auf einfach zu montierende und sichere Weise befestigt, sondern die Befestigungsanordnung vermeidet auch eine unerwünschte Schwächung des Koppelstücks.

Die Erfindung wird nachfolgend unter Bezugnahme auf die beigefügte Zeichnung näher erläutert, in der ein vorteilhaftes Ausführungsbeispiel dargestellt ist. Es zeigen:
- Fig. 1: eine Rückansicht einer Gelenkprothese in einer teilweise geschnittenen Darstellung,

- Fig. 2: eine vergrößerte Rückansicht des Koppelstücks mit einer angrenzenden Komponente;
- Fig. 3: eine Detailansicht der Lagerbuchse von der Seite in geschnittener Darstellung;

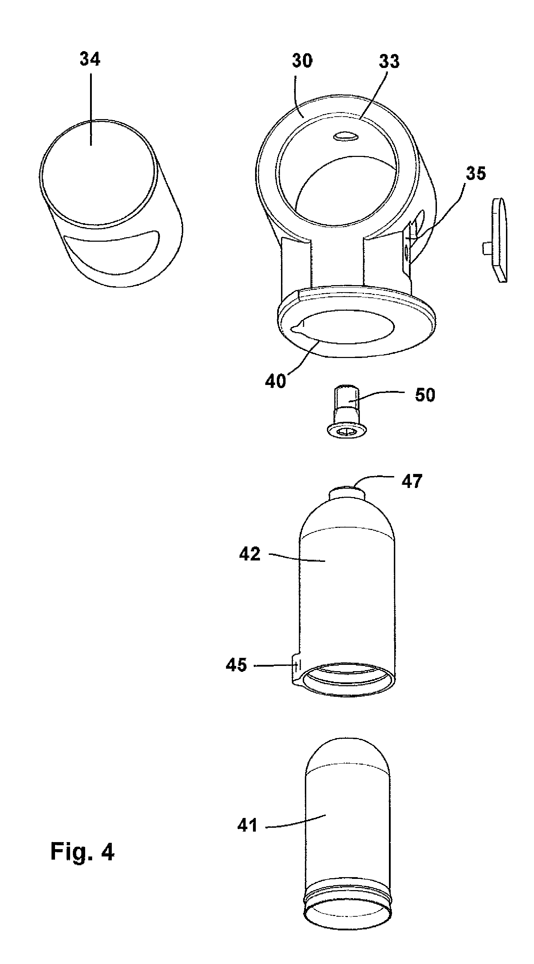

- Fig. 4: eine Explosionsansicht Kopplungsstücks mit der Lagerbüchse;

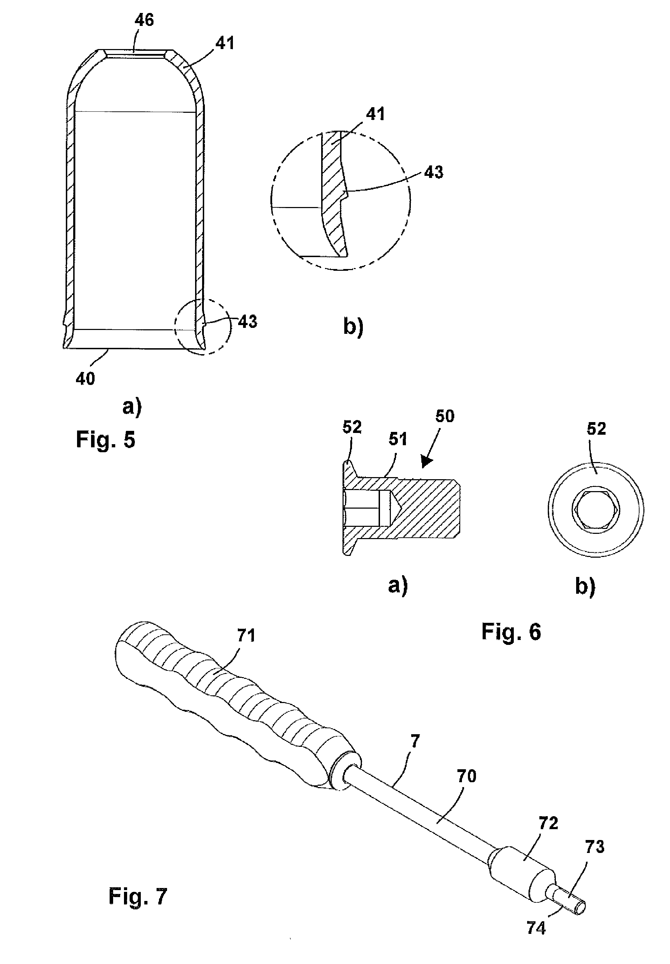

- Fig. 5 a, b: Detailansichten bei Rasteinrichtung;
- Fig. 6 a, b: Detailansicht eines Sicherungselements; und
- Fig. 7: ein Extraktor.

Die Endoprothese gemäß einem Ausführungsbeispiel der Erfindung wird anhand einer Kniegelenkendoprothese erläutert.

Die Endoprothese eines Kniegelenks besteht im Wesentlichen aus zwei Komponenten 1, 2, von denen eine als tibiale Komponente 1 und die andere als femorale Komponente 2 ausgebildet ist. Bei der femoralen Komponente 2 schließt sich an einen Stiel 20, der in einen Oberschenkelknochen eines Patienten eingesetzt wird, eine femorale Lagerhälfte 21 an, welche über zwei gabelartig zur tibialen Komponente 1 vorspringende kondylenartige Gleitkufen 22 verfügt. Diese stützen sich auf einem Tibiaplateau 12 ab, das auf einer tibialen Lagerhälfte 11 angeordnet ist, welche über einen Stiel 10 an einem Unterschenkelknochen des Patienten befestigt ist.

Dazwischen ist ein Koppelstück 3 angeordnet, welches ein T-Stück 30 als Hauptkörper mit einem in seinem oberen Bereich angeordneten Aufnahmeauge 33 für einen Achsbolzen 34 und einer Lagerbuchse 32 für eine sich aus dem Tibiaplateau 11 erhebenden Lagerzapfen 31 aufweist.

Das erste Lager (Flexionslager) ermöglicht eine Schwenkbewegung zwischen den Komponenten 1 und 2, realisiert also eine Beugebewegung zwischen Ober- und Unterschenkel. Diese Schwenkbewegung um die Achse des Achsbolzens 34 bildet damit die erste Achse für die Bewegung der Knieendogelenkprothese. Quer zu dieser Achse ist der Zapfen 31 ausgerichtet, welche eine zweite Achse bildet für eine Rotationsbewegung, mit der sich die femorale Komponente 2 relativ zur tibialen Komponente 1 um die zweite Achse verdreht.

Für dieses Rotationslager ragt der Zapfen 31 in die Lagerbuchse 32 an dem Koppelstück 3 hinein. Zwischen den beiden ist ein Lagereinsatz 4 angeordnet. Dieser ist im Aufnahmeauge 33 über ein an seinem oberen Ende befindliches Sicherungselement 5 in Gestalt einer Schraube 50 befestigt.

Der Lagereinsatz 4 ist von einer im Wesentlichen zylindrischen Grundform und weist an seinem oberen Ende einen halbkugelartigen Dom auf. Der Lagereinsatz 4 besteht aus einer Gleithülse 41 und einer Stützhülse 42. Der Außendurchmesser der Lagerhülse 41 ist dabei so groß gewählt, dass sich ein leichter Presssitz in der Innenwandung der Stützhülse 42 ergibt. An ihrem unteren Ende weist die Stützhülse 41 eine Mündung 40 für den Zapfeneintritt auf. An der Innenseite ist ein Fixationselement 43 über den Umfang umlaufend angeordnet, wobei es vorzugsweise kontinuierlich ist, aber auch mehrfach unterbrochen sein kann. Es greift in einen entsprechend ausgeformten Hinterschnitt (gesehen von der Mündung 40 des Lagereinsatzes 4) und sichert so die Gleithülse 41 an einem Auswandern aus der Stützhülse 42. Die Stützhülse 42 hat an ihrem mündungsseitigen Ende an ihrer Außenseite eine radial vorspringende Leiste 45, die in eine komplementär geformte Ausnehmung an der Innenseite der Lagerbuchse 32 des Koppelstücks 3 formschlüssig eingreift. Damit ist die Stützhülse 42 an einer unerwünschten Rotation gegenüber dem Kopplungsstück 3 gehindert.

Die Komponenten des Lagereinsatzes 4, also die Stützhülse 42 sowie die Gleithülse 41 erstrecken sich bis zur Mündung 40 des Lagereinsatzes 4, d.h. beide bilden mit ihrer unteren Stirnfläche die Berandung der Mündung 40. Die Gleithülse 41 kann an der Innenseite der Gleithülse 41 eine konisch sich verjüngende Form vorgesehen sein. Sie erleichtert das Einsetzen des Zapfens 31 in den Lagereinsatz 4.

An seinem oberen Ende weist die Gleithülse 41 eine Zugangsöffnung 46 auf. Sie ist mit einer kegeligen Randfläche versehen, deren Weite nach oben hin vergrößert. Mit ihr fluchtend sind in der Stützhülse 42 eine erste Bohrung 47 und in dem Aufnahmeauge 33 eine zweite Bohrung 38 vorgesehen, welche mit einem Innengewinde 39 versehen ist. In der Bohrung 47 der Stützhülse 42 ist ein Innengewinde 48 angeordnet. In diesen Bohrungen ist die Schraube 50 als Sicherung eingesetzt und mit ihrem Schaft 51 in das Innengewinde 39 geschraubt. Der Kopf 52 der Schraube 50 weist einen größeren Durchmesser als die Weite der Zugangsöffnung 46. Sie liegt fest auf der Innenseite der Stützhülse 42 auf und sichert sie in ihrer Position.

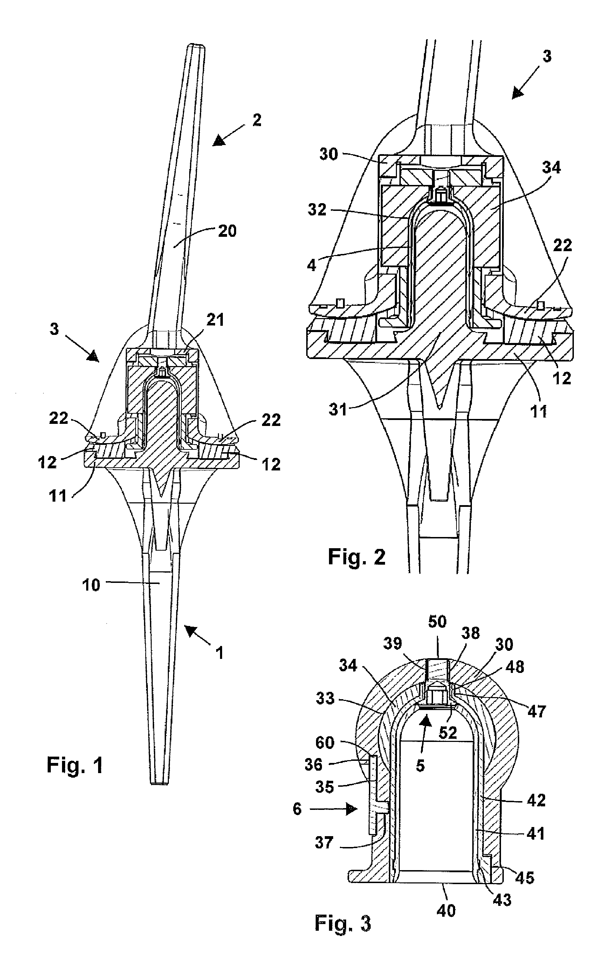

An seiner Vorderseite (in der Darstellung in Figur 3 rechts) ist das Koppelstück 3 mit einer planen Aufnahmefläche 35 versehen. An ihrem oberen Ende ist eine Tasche 36 in dem Aufnahmeauge 33 ausgeformt, die an ihrem Grund plan in die Aufnahmefläche 35 übergeht. Im mittleren Bereich der Aufnahmefläche 35 ist eine Halteöffnung 37 vorgesehen, die als Durchgangsöffnung in der Lagerbuchse 31 bis zur Stützhülse 42 ausgeführt ist. Auf die Aufnahmefläche 35 aufgesetzt und mit ihrer oberen Kante in die Tasche 36 eingeschoben ist eine Prallschutzplatte 6. Durch das Einschieben in die Tasche 36 mit ihrer oberen Kante 60 ist sie gegenüber einem Abheben von der Aufnahmefläche 35 gesichert, insbesondere unter Krafteinwirkung von vorne (in Figur 3 von rechts) beim Erreichen der Anschlagsposition. Um ein Verschieben der Prallschutzplatte 6 insbesondere nach unten zu vermeiden, ist an ihrer Rückseite ein Vorsprung 62 ausgeformt, welcher formschlüssig in die Halteöffnung 37 eingreift und somit die Prallschutzplatte 6 in ihrer Position sichert.

Weiter ist ein Extraktor 7 vorgesehen, der als ein Schraubendreher mit einem Schaft 70 an dem Handgriff 71 an seinem hinteren Ende ausgeführt ist. Am vorderen Ende ist eine Führungstonne 72 angeordnet, die einen größeren Durchmesser als der Schaft 70 aufweist, und deren Durchmesser auf die Innenweite des Lagereinsatzes 4 abgestimmt ist. Unter abgestimmt wird hierbei verstanden, dass sie etwa 1 mm Untermaß aufweist, so dass sich eine Spielpassung ergibt. Im vorderen Bereich der Führungstonne 72 ist eine Schraubspitze 73 angeordnet, die ein Außengewinde 74 aufweist. Es ist so ausgeführt, dass es in das Innengewinde 48 in der Bohrung 47 fasst.

Im montierten Zustand ist der Lagereinsatz 4 durch Eindrehen der Sicherungsschraube 50 der Sicherung 5 fixiert. Dabei liegt die Sicherungsschraube 50 mit ihrem Kopf 52 auf der Innenseite der Stützhülse 42 an und zieht diese gegen den im Achsauge 33 gelagerten Achsbolzen 34. Der Lagereinsatz 4 ist damit an einer Bewegung aus der Lagerbuchse 32 gesichert.

Zur Demontage wird, nachdem der Zapfen 31 aus der Lagerbuchse 32 ausgezogen wurde, die Sicherungsschraube 50 in an sich bekannter Weise mittels eines Schraubendrehers (nicht dargestellt) gelöst. Hierbei drückt die Schraube 50 mit ihrem Kopf 52, da er eine größere Weite als die Durchgangsöffnung 46 in der Gleithülse 41 aufweist, die Gleithülse 41 nach unten aus der Stützhülse 42 heraus. Das Herausdrücken kann unterstützt werden durch ein Zusammendrücken der Gleithülse 41 im Bereich der Mündung 40, um so ein Ausrasten der Rastelemente 43, 44 zu erleichtern. Im vielen Fällen kann dann die Stützhülse 42 aus der Lagerbuchse 32 entnommen werden. Sitzt sie jedoch fest, wie es insbesondere nach Überlastung oder bei langer Einsatzdauer leicht auftreten kann, so kann nach Entfernen der Sicherungsschraube 50 der Extraktor 7 mit der Führungstonne 72 in den Innenraum der Stützhülse 42 eingebracht werden, und Extraktor 7 mit seinem Gewinde 74 an der Schraubspitze 73 in das Innengewinde 48 der Stützhülse 42 eingeschraubt werden. Damit ist der Extraktor 7 zugfest mit der Stützhülse 42 verbunden und sie kann aus der Lagerbuchse 32 herausgezogen werden.

Dank der Erfindung ist nur ein verhältnismäßig kleines Zugangsloch in Gestalt der Bohrung 38 im Koppelstück erforderlich. Das Koppelstück kann im Übrigen massiv ausgeführt sein, d.h. insbesondere im Bereich des Aufnahmeauges 33 sind keine weiteren Durchbrechungen erforderlich. Damit wird eine Schwächung des Aufnahmeauges 33 vermieden. Gleichzeitig ermöglicht die Erfindung eine sichere Befestigung des Lagereinsatzes 4 und ein einfaches Herausnehmen. Durch die Anordnung der Prallschutzplatte 6 in der Tasche 36 wird ebenfalls eine Schwächung vermieden, wie sie im Stand der Technik durch eine große Anzahl von Haftlöchern im Bereich der Aufnahmefläche 35 aufgetreten ist.

## Patentansprüche

1. Gelenkprothese mit einer distalen Komponente (1) zur Verankerung an einem ersten Knochen, einer proximalen Komponente (2) zur Verankerung mit einem zweiten Knochen, einem Kopplungsstück (3), das der ersten Komponente (1) ein Flexionslager um eine erste Achse (34) und mit der zweiten Komponente (2) ein aus Zapfen (31) und Lagerbuchse (32) gebildetes Rotationslager um eine quer zur ersten Achse (34) orientierte zweite Achse bildet, wobei das Rotationslager einen mehrlagigen Lagereinsatz (4) umfasst mit einer den Zapfen (31) umfassenden Gleithülse (41) und eine diese umkammernde Stützhülse (42), die über ein Sicherungselement (5) an dem Koppelstück (3) befestigt ist
**dadurch gekennzeichnet, dass**
das Sicherungselement (5) ein Betätigungsorgan (52) innerhalb der Stützhülse (42) aufweist und über zwei fluchtende Bohrungen (47, 38) in Schutzhülse (42) und Koppelstück (3) zugfest mit dem Koppelstück (3) verbindbar ist.

2. Rotationsgelenkprothese nach Anspruch 1, **dadurch gekennzeichnet, dass** an der Gleithülse (41) die Zugangsöffnung (46) zum Betätigungsorgan (52) vorgesehen ist.

3. Rotationsgelenkprothese nach Anspruch 2, **dadurch gekennzeichnet, dass** das Betätigungsorgan (52) eine grö-βere Weite aufweist als die Zugangsöffnung (46).

4. Rotationsgelenkprothese nach einem der vorangehenden Ansprüche, **dadurch gekennzeichnet, dass** das Sicherungselement (5) eine Schraube (50) umfasst, deren Kopf das Betätigungsorgan (52) bildet.

5. Rotationsgelenkprothese nach einem der vorangehenden Ansprüche, **dadurch gekennzeichnet, dass** Bohrungen (47, 38) gestufte Weiten aufweisen.

6. Rotationsgelenkprothese nach einem der vorangehenden Ansprüche, **dadurch gekennzeichnet, dass** die Zugangsöffnung (46) eine größere Weite als der Kopf (52) aufweist, der sich vorzugsweise nach innen verjüngt.

7. Rotationsgelenkprothese nach einem der Ansprüche 2 bis 6, **dadurch gekennzeichnet, dass** an der Stützhülse (42) eine zu der Zugangsöffnung (46) fluchtende Aufnahme (48) für einen Extraktor (7) vorgesehen ist.

8. Rotationsgelenkprothese nach Anspruch 7, **dadurch gekennzeichnet, dass** die Aufnahme als ein Innengewinde ausgeführt ist.

9. Rotationsgelenkprothese nach einem der vorangehenden Ansprüche, **dadurch gekennzeichnet, dass** die Gleithülse (41) über ein im Bereich ihrer Aufnahmeöffnung (70) angeordnete Fixationselement (43), welches in einen Hinterschnitt (44) an der Innenfläche der Stützhülse (42) greift, vor einer axialen Bewegung gesichert ist.

10. Rotationsgelenkprothese nach einem der vorangehenden Ansprüche, **dadurch gekennzeichnet, dass** an der Außenseite der Stützhülse (42) eine radial vorspringende Nase (45) vorgesehen ist, die in einem komplementär geformten Rezess an dem Kopplungsstück (3) greift.

11. Rotationsgelenkprothese nach einem der vorangehenden Ansprüche, **dadurch gekennzeichnet, dass** die Lagerbuchse (32) glattwandig mit vorzugsweise zylindrischer Innenform ausgeführt ist.

12. Rotationsgelenkprothese nach einem der vorangehenden Ansprüche, **dadurch gekennzeichnet, dass** an der Vorderseite des Koppelstücks (3) eine Aufnahmefläche (35) für einen Prallschutz (6) vorgesehen ist, zu deren Verlängerung eine taschenartige Ausnehmung ausgebildet ist.

13. Rotationsgelenkprothese nach Anspruch 12, **dadurch gekennzeichnet, dass** die taschenartige Ausnehmung (36) einen rechteckigen Querschnitt aufweist und plan in die Aufnahmefläche (35) übergeht.

14. Rotationsgelenkprothese nach einem der Ansprüche 12 oder 13, **dadurch gekennzeichnet, dass** maximal zwei Vertiefungen (37) als Verschiebesicherung für den Prallschutz (6) vorgesehen sind.
